(19) Europäisches Patentamt
European Patent Office
Office européen des brevets

(11) Veröffentlichungsnummer: **0 130 938**
**B1**

(12) # EUROPÄISCHE PATENTSCHRIFT

(45) Veröffentlichungstag der Patentschrift: **15.02.89**

(51) Int. Cl.⁴: **C 07 D 249/20**

(21) Anmeldenummer: **84810259.6**

(22) Anmeldetag: **25.05.84**

(54) **Verfahren zur Herstellung von 2-Arylbenzotriazolen.**

(30) Priorität: **01.06.83 CH 2986/83**

(43) Veröffentlichungstag der Anmeldung: **09.01.85 Patentblatt 85/2**

(45) Bekanntmachung des Hinweises auf die Patenterteilung: **15.02.89 Patentblatt 89/7**

(84) Benannte Vertragsstaaten: **DE FR GB IT**

(56) Entgegenhaltungen:
**DE-A-2 835 529**
**JP-A-63 037 978**

**PATENTS ABSTRACTS OF JAPAN, Band 9, Nr. 28 (C-264) 1751, 6. Februar 1985; & JP - A - 59 172 481 (SHIYUUICHI SEINO) 29.09.1984**
**PATENTS ABSTRACTS OF JAPAN, Band 9, Nr. 21 (C-263) 1744, 29. Januar 1985; & JP - A - 59 170 172 (SHIYUUICHI SEINO) 26.09.1984**
**PATENTS ABSTRACTS OF JAPAN, Band 7, Nr. 11 (C-145) 1156, 18. Januar 1983; JP - A - 57 167 976 (SHIPURO KASEI K.K.) 16.10.1982**

(73) Patentinhaber: **CIBA- GEIGY AG, Klybeckstrasse 141, CH- 4002 Basel (CH)**

(72) Erfinder: **Davatz, Alexander, Dr., Gstaltenstrasse 9, CH- 4416 Bubendorf (CH)**
Erfinder: **Somlo, Tibor, Dr., Florastrasse 8, CH- 4127 Birsfelden (CH)**

EP 0 130 938 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein Verfahren zur Herstellung von 2-(2'-Hydroxyphenyl)-benztriazolen aus 2-Nitro-2'-hydroxyazobenzolen.

Die Reduktion von 2-Nitro-2'-hydroxyazobenzolen zu 2-(2'-Hydroxyphenyl)-benztriazolen in basischalkoholischem Medium ist bekannt. Nebst einer aromatischen Dihydroxy- oder Dioxo-Verbindung als Katalysator werden aber Reduktionsmittel wie Zink, Ammoniumsulfid, Alkalisulfid, Ammoniumhydrogensulfid, Alkalihydrogensulfid, Alkalidithionit oder Hydrazinhydrat benötigt (JP-A-63379/78).

Doch ist es aufwendig, die bei der Reduktion mit Metallen wie Zink anfallenden Oxide zu beseitigen und zudem zu verhindern, dass sie die Abwässer belasten. Die schwefelhaltigen Reduktionsmittel sind den Metallen unterlegen und hinterlassen in den Produkten schwer entfernbare Schwefelverunreinigungen. Hydrazinhydrat ist wohl ein potentes Reduktionsmittel, seine Handhabung stellt aber wegen der hohen Toxizität ein Sicherheitsrisiko dar.

In den bisher beschriebenen Verfahren zur Reduktion von 2-Nitro-2'-hydroxyazobenzolen zu 2-(2'-Hydroxyphenyl)-benztriazolen wirkten Alkohole, soweit solche verwendet wurden, als Lösungsmittel. Zudem werden nach bisheriger allgemeiner Erfahrung Nitrogruppen von basischen Alkohol-Lösungen nicht angegriffen (siehe ORGANIKUM, VEB Deutscher Verlag der Wissenschaften, Berlin 1976, Seite 604). Auf der Suche nach einem wirtschaftlicheren und umweltfreundlicheren Verfahren wurden nun Reaktionsbedingungen gefunden, unter denen sich 2-Nitro-2'-hydroxyazobenzole mit basischen Alkohol-Lösungen, deren Alkohol mehr als ein Kohlenstoffatom hat zu 2-(2'-Hydroxyphenyl)-benztriazolen doch reduzieren lassen.

Die vorliegende Erfindung betrifft somit ein Verfahren zur Herstellung von 2-(2'-Hydroxyphenyl)-benztriazolen durch Reduktion von 2-Nitro-2'-hydroxyazobenzolen in einem stark basischen Medium und in Gegenwart von aromatischen Benzodiolen, Naphthodiolen, Benzochinonen oder Naphthochinonen, dadurch gekennzeichnet, dass man die Reduktion mit einem bei Raumtemperatur flüssigen Alkohol mit mehr als einem Kohlenstoffatom ohne Mitverwendung eines weiteren Reduktionsmittels durchführt.

Bevorzugt werden nach dem erfindungsgemässen Verfahren aus Verbindungen der Formel II Verbindungen der Formel I hergestellt,

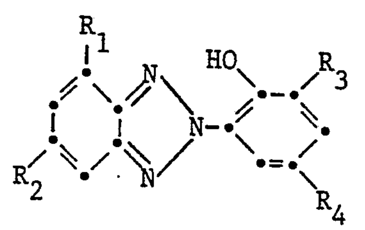

I

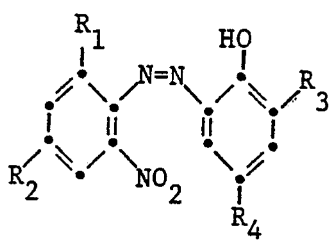

II

in denen

$R_1$ Wasserstoff oder Halogen,
$R_2$ Wasserstoff, Alkyl, Alkoxy oder Halogen,
$R_3$ Wasserstoff, Alkyl, Cycloalkyl, Aralkyl oder Aryl,
$R_4$ Wasserstoff, Alkyl, Cycloalkyl, Aralkyl, Aryl oder Alkoxy

bedeuten.

In den früheren Verfahren hatte der Alkohol, wo solcher verwendet wurde, nur Lösungsmittelfunktion. Es musste jedesmal zusätzlich ein Reduktionsmittel zugegeben werden. Im erfindungsgemässen Verfahren dagegen übt der eingesetzte Alkohol eine Doppelfunktion aus, d. h. er wirkt als Lösungsmittel und Reduktionsmittel. Dadurch wird gegenüber den früheren Verfahren ein Rohstoff eingespart. Da zudem keine Reduktionsmittel mehr wie Zink, Sulfidm Hydrogensulfid, Dithionit oder Hydrazinhydrat benötigt werden, fallen auch die geschilderten Nachteile bezüglich Arbeitsaufwand, Umwelt und Sicherheit weg. Langwierige Trennprozesse, insbesondere im Fall der metallischen Reduktionsmittel, werden überflüssig. Am Ende des Prozesses fällt das Reaktionsprodukt jeweils aus und lässt sich leicht isolieren. Die Ausbeuten sind hoch. Das neue Verfahren ist somit wirtschaftlicher und umweltfreundlicher.

Besonders bevorzugt werden nach dem erfindungsgemässen Verfahren aus Verbindungen der Formel II Verbindungen der Formel I hergestellt,

in denen

$R_1$ Wasserstoff oder Chlor,
$R_2$ Wasserstoff, Alkyl mit 1 bis 5 Kohlenstoffatomen, Alkoxy mit 1 bis 4 Kohlenstoffatomen oder Chlor,
$R_3$ Wasserstoff, Alkyl mit 1 bis 12 Kohlenstoffatomen, Cycloalkyl mit 5 bis 8 Kohlenstoffatomen, Aralkyl mit 7 bis 9 Kohlenstoffatomen oder Phenyl,
$R_4$ Wasserstoff, Alkyl mit 1 bis 12 Kohlenstoffatomen, Cycloalkyl mit 5 bis 8 Kohlenstoffatomen, Aralkyl mit 7 bis 9 Kohlenstoffatomen, Phenyl oder Alkoxy mit 1 bis 12 Kohlenstoffatomen bedeuten.

In der Bedeutung von Halogen können $R_1$ und $R_2$ Chlor bedeuten. Ist $R_2$ ein Alkyl, so handelt es sich beispielsweise um Methyl, Äthyl, Propyl, Isopropyl, n-Butyl, tert.-Butyl, Amyl oder tert.-Amyl.

Bedeutet $R_2$ ein Alkoxy, so kann es sich zum Beispiel um Methoxy, Äthoxy oder n-Butoxy handeln.

Sind $R_3$ oder $R_4$ Alkyl, so handelt es sich beispielsweise um Methyl, Äthyl, sek.-Butyl, tert.-Butyl, tert.-Amyl, tert.-Octyl oder n-Dodecyl.

Bedeuten $R_3$ oder $R_4$ Cycloalkyl, so kann es sich zum Beispiel um Cyclopentyl, Cyclohexyl oder Cyclooctyl handeln.

Sind $R_3$ oder $R_4$ Aralkyl, so handelt es sich beispielsweise um Benzyl, α-Methylbenzyl oder α,α-Dimethylbenzyl.

In der Bedeutung von Aryl, können $R_3$ oder $R_4$ Phenyl sein.

Ist $R_4$ ein Alkoxy, so kann es sich zum Beispiel um Methoxy, Äthoxy, Propoxy, Butoxy, Pentyloxy, iso-Butoxy, Octoxy oder Dodecyloxy handeln.

Typische Verbindungen der Formel I sind in den Beispielen dargestellt.

Für das erfindungsgemässe Verfahren eignen sich insbesondere primäre Alkanole, sekundäre Alkanole, Aralkanole, Glykole und Glykolmonoalkyläther. Bevorzugte primäre Alkanole sind beispielsweise Äthanol, n-Propanol, n-Butanol oder n-Octylalkohol. Bevorzugte sekundäre Alkanole sind zum Beispiel Isopropanol, sek.-Butanol oder sek.-Octanol. Bevorzugtes Aralkanol ist Benzylalkohol. Bevorzugte Glykole sind zum Beispiel 1,2-Äthandiol, 1,3-Propandiol oder 1,4-Butandiol. Bevorzugte Glykolmonoalkyläther sind beispielsweise Äthylenglykolmonomethyläther, Äthylenglykolmonoäthyläther und Äthylenglykolmonobutyläther. Besonders bevorzugt werden die sekundären Alkanole. Ganz besonders bevorzugt sind Isopropanol und sek.-Butanol.

Im erfindungsgemässen Verfahren kann das stark basische Medium durch Zugabe eines Alkalihydroxides wie Natrium- oder Kaliumhydroxid, vorzugsweise Natriumhydroxid, oder eines Alkalimetalles wie Natrium oder Kalium, vorzugweise Natrium, zu einem oben defininierten Alkohol erzeugt werden. Dabei können die erwähnten Alkalihydroxide bzw. Alkalimetalle in mindestens ein, vorzugsweise zwei Moläquivalenten bezüglich des zu reduzierenden Substrates eingesetzt werden.

Als Katalysatoren wirken im erfindungsgemässen Verfahren Benzodiole, Naphthodiole, Benzochinone oder Naphthochinone. Als Diole kommen z. B. 1,2-Benzodiole und 1,4-Benzodiole oder 1,2-Naphthodiole, 1,4-Naphthodiole und 2,6-Naphthodiole in Frage. Die aromatischen Kerne können unsubstituiert oder ganz oder teilweise z. B. mit Alkyl oder Halogen substituiert sein. Bevorzugt sind unsubstituierte Benzodiole.

Die Benzochinone können unsubstituiert oder ganz oder teilweise z. B. mit Alkyl, wie Methyl, oder Halogen wie Chlor, substituiert sein. Benzochinone sind z. B. 1,2-Benzochinone und 1,4-Benzochinone. Bevorzugt im erfindungsgemässen Verfahren werden unsubstituierte oder substituierte Naphthochinone, wie 1,2-Naphthochinone, 1,4-Naphthochinone oder 2,6-Naphthochinone eingesetzt. Sind die Kerne substituiert, so sind sie es z. B. mit Halogen, Hydroxyl, Alkyl, Dialkylamino, Piperidino oder Morpholino. Besonders bevorzugt sind 1,4-Naphthochinone der Formel III

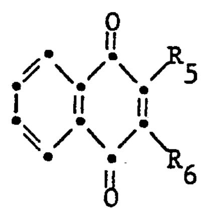
III

worin
$R_5$ Wasserstoff, Halogen oder Hydroxyl,
$R_6$ Wasserstoff, Halogen, Alkyl, Dialkylamino, Piperidino oder Morpholino bedeuten.

Vor allem bevorzugt sind Verbindungen der Formel III, in denen
$R_5$ Wasserstoff, Chlor oder Hydroxyl,
$R_6$ Wasserstoff, Chlor, Alkyl mit 1 bis 12 Kohlenstoffatomen, Dialkylamino mit 2 bis 24 Kohlenstoffatomen, Piperidino oder Morpholino bedeuten.

Bedeutet $R_6$ ein Alkyl, so handelt es sich beispielsweise um Methyl, Äthyl, n-Butyl, sek.-Butyl, n-Octyl oder n-Dodecyl.

Bedeutet $R_6$ Dialkylamino, so kann es sich z. B. um Dimethylamino, Diäthylamino, Dibutylamino, Dioctylamino oder Didodecylamino handeln.

Ganz besonders bevorzugt im erfindungsgemässen Verfahren sind 1,4-Naphtochinon, 2,3-Dichlornaphtochinon, 2-Chlornaphtochinon, 2-Hydroxy-3-methylnaphtochinon oder 2-Chlor-3-dimethylaminonaphtochinon.

Die als Katalysator wirkenden aromatischen Diole und Chinone können, bezogen auf das zu reduzierende Substrat, beispielsweise in Mengen von 1 - 20 Mol-%, insbesondere 5 - 10 Mol-% eingesetzt werden.

Das erfindungsgemässe Verfahren kann z. B. bei etwa 20°C bis etwa 120°C, insbesondere bei etwa 40°C bis etwa 100°C, und vor allem bei etwa 50°C bis etwa 80°C durchgeführt werden.

Im erfindungsgemässen Verfahren soll der Wassergehalt des Reaktionsmediums zweckmässigerweise kleiner als 15 Vol-% sein. Bevorzugt wird die Reduktion in einem wasserfreien Medium durchgeführt.

Die Verbindungen der Formel I sind bekannte Stabilisatoren für organische Materialien, wie zum Beispiel für organische Polymere. Die Verbindungen der Formel II sind als Ausgangsstoffe bekannt.

In den folgenden Beispielen wird die Erfindung näher beschrieben.

**Beispiel 1:**

Ein 750 ml Sulfierkolben wird mit 220 g sek.-Butanol, 34,7 g 2-Nitro-4-chlor-2'-hydroxy-3'-tert.-butyl-5-methylazobenzol, 2,3 g 2,3-Dichlornaphrochinon und 26 g 50-gewichtsprozentiger wässeriger Natronlauge beschickt. Das Gemisch wird während zwei Stunden bei 40 - 45°C und während drei Stunden bei 70 - 75°C gerührt. Man tropft 500 ml Wasser zu und filtriert die ausgefallenen Kristalle ab. Man wäscht sie mit Wasser und trocknet sie im Vakuum bei 60 - 70°C. Man erhält 2-tert.-Butyl-4-methyl-6-(4'-chlor-2'-benztriazolyl)-phenol in hoher Ausbeute.

**Beispiel 2:**

Ein 750 ml Sulfierkolben wird mit 300 g Cyclohexanol, 35,5 g 2-Nitro-2'-hydroxy-3',5'-bis-tert.-butyl-azobenzol, 2,3 g 2,3-Dichlornaphtochinon und 25,6 g Natriumhydroxid beschickt. Man rührt das Gemisch während drei Stunden bei 70 - 75°C und stellt mit 5-volumen-prozentiger wässeriger Salzsäure den pH-Wert der Lösung auf 1,5. Die wässerige Phase wird abgetrennt und verworfen. Die organische Phase wäscht man mit Wasser und kühlt sie auf -5°C ab. Es kristallisiert 2,4-Di-tert.-butyl-6-(2'-benztriazolyl)-phenol in hoher Ausbeute aus.

**Patentansprüche**

1. Verfahren zur Herstellung von 2-(2'-Hydroxyphenyl)-benztriazolen durch Reduktion von 2-Nitro-2'-hydroxyazobenzolen in einem stark basischen Medium und in Gegenwart von aromatischen Benzodiolen, Naphthodiolen, Benzochinonen oder Naphthochinonen, dadurch gekennzeichnet, dass man die Reduktion mit einem bei Raumtemperatur flüssigen Alkohol mit mehr als einem Kohlenstoffatom ohne Mitverwendung eines weiteren Reduktionsmittels durchführt.

2. Verfahren nach Anspruch 1, dadurch gekennzeichnet, dass man aus Verbindungen der Formel II Verbindungen der Formel I herstellt,

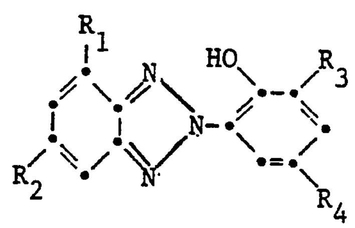

I

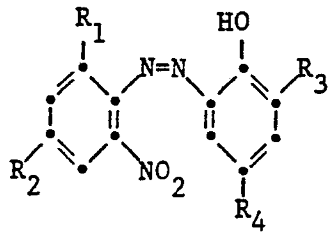

II

in denen

- $R_1$ Wasserstoff oder Halogen,
- $R_2$ Wasserstoff, Alkyl, Alkoxy oder Halogen,
- $R_3$ Wasserstoff, Alkyl, Cycloalkyl, Aralkyl oder Aryl,
- $R_4$ Wasserstoff, Alkyl, Cycloalkyl, Aralkyl, Aryl oder Alkoxy

bedeuten.

3. Verfahren nach Anspruch 1, dadurch gekennzeichnet, dass man aus Verbindungen der Formel II Verbindungen der Formel I herstellt, in denen

- $R_1$ Wasserstoff oder Chlor,
- $R_2$ Wasserstoff, Alkyl mit 1 bis 5 Kohlenstoffatomen, Alkoxy mit 1 bis 4 Kohlenstoffatomen oder Chlor,
- $R_3$ Wasserstoff, Alkyl mit 1 bis 12 Kohlenstoffatomen, Cycloakyl mit 5 bis 8 Kohlenstoffatomen, Phenyl oder Aralkyl mit 7 bis 9 Kohlenstoffatomen,
- $R_4$ Wasserstoff, Alkyl mit 1 bis 12 Kohlenstoffatomen, Cycloalkyl mit 5 bis 8 Kohlenstoffatomen, Aralkyl mit 7 bis 9 Kohlenstoffatomen, Phenyl oder Alkoxy mit 1 bis 12 Kohlenstoffatomen bedeuten.

4. Verfahren nach Anspruch 1, dadurch gekennzeichnet, dass die Alkalihydroxide bzw. Alkalimetalle, bezogen auf das zu reduzierende Substrat, in Mengen von mindestens 1 Moläquivalent eingesetzt werden.

5. Verfahren nach Anspruch 1, dadurch gekennzeichnet, dass der Alkohol Isopropanol oder sek.-Butanol ist.

6. Verfahren nach Anspruch 1, dadurch gekennzeichnet, dass die Reduktion in Gegenwart eines unsubstituierten oder substituierten Benzodiols oder eines unsubstituierten oder substituierten Naphthochinons durchgeführt wird.

7. Verfahren nach Anspruch 1, dadurch gekennzeichnet, dass man die Reduktion in einem Medium durchführt, das kein oder maximal 15 Vol-% Wasser enthält.

**Claims**

1. A process for preparing 2-(2′-hydroxyphenyl)-benzotriazoles by reducing 2-nitro-2′-hydroxyazobenzenes in a strongly basic medium and in the presence of aromatic benzodiols, naphthodiols, benzoquinones or naphthoquinones, which comprises carrying out the reduction with an alcohol which has more than one carbon atom, and is liquid at room temperature without the additional use of a further reducing agent.

2. The process according to claim 1, wherein there are prepared, from compounds of the formula II, compounds of the formula I

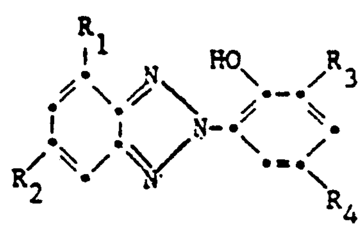

I

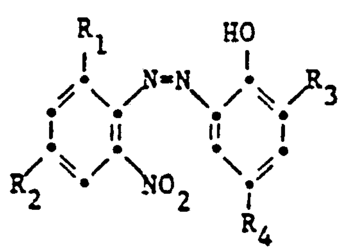

II

in which

- $R_1$ is hydrogen or halogen,
- $R_2$ is hydrogen, alkyl, alkoxy or halogen,
- $R_3$ is hydrogen, alkyl, cycloalkyl, aralkyl or aryl, and
- $R_4$ is hydrogen, alkyl, cycloalkyl, aralkyl, acyl or alkoxy.

3. The process according to claim 1, wherein there are prepared, from compounds of the formula II, compounds of the formula I in which
$R_1$ is hydrogen or chlorine,
$R_2$ is hydrogen, alkyl having 1 to 5 carbon atoms, alkoxy having 1 to 4 carbon atoms or chlorine,
$R_3$ is hydrogen, alkyl having 1 to 12 carbon atoms, cycoakyl (sic) having 5 to 8 carbon atoms, phenyl or aralkyl having 7 to 9 carbon atoms, and
$R_4$ is hydrogen, alkyl having 1 to 12 carbon atoms, cycloalkyl having 5 to 8 carbon atoms, aralkyl having 7 to 9 carbon atoms, phenyl or alkoxy having 1 to 12 carbon atoms.

4. The process according to claim 1, wherein the alkali metal hydroxides or alkali metals are used in amounts of at least 1 mole equivalent relative to the substrate to be reduced.

5. The process according to claim 1, wherein the alcohol is isopropanol or sec.-butanol.

6. The process according to claim 1, wherein the reduction is carried out in the presence of an unsubstituted or substituted benzodiol or an unsubstituted or substituted naphthoquinone.

7. The process according to claim 1, wherein the reduction is carried out in a medium which contains at most 15 % by volume of water, if any.

**Revendications**

1. Procédé de préparation d'(hydroxy-2 phényl)-2 benzotriazoles par réduction de nitro-2 hydroxy-2' azobenzènes dans un milieu très basique et en présence de benzodiols, de naphtodiols, de benzoquinones ou de naphtoquinones aromatiques, procédé caractérisé en ce qu'on effectue la réduction avec un alcool qui contient plus d'un atome de carbone et qui est liquide à la température ambiante, sans avoir recours à un réducteur supplémentaire.

2. Procédé selon la revendication 1 caractérisé en ce qu'on prépare des composés de formule I à partir de composés de formule II:

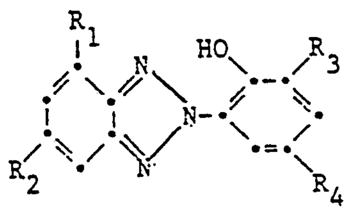
I

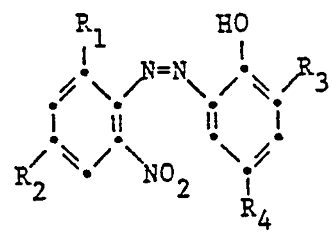
II

formules dans lesquelles:
$R_1$ représente l'hydrogène ou un halogène,
$R_2$ représente l'hydrogène, un alkyle, un alcoxy ou un halogène,
$R_3$ représente l'hydrogène, un alkyle, un cycloalkyle, un aralkyle ou un aryle et
$R_4$ représente l'hydrogène, un alkyle, un cycloalkyle, un aralkyle, un aryle ou un alcoxy.

3. Procédé selon la revendication 1 caractérisé en ce qu'on prépare des composés de formule I à partir de composés de formule II dans lesquels:
$R_1$ représente l'hydrogène ou le chlore,
$R_2$ représente l'hydrogène, un alkyle contenant de 1 à 5 atomes de carbone, un alcoxy en $C_1$-$C_4$ ou le chlore,
$R_3$ représente l'hydrogène, un alkyle en $C_1$-$C_{12}$, un cycloalkyle en $C_5$-$C_8$, un phényle ou un aralkyle en $C_7$-$C_9$ et
$R_4$ représente l'hydrogène, un alkyle en $C_1$-$C_{12}$, un cycloalkyle en $C_5$-$C_8$, un aralkyle en $C_7$-$C_9$, un phényle ou un alcoxy en $C_1$-$C_{12}$.

4. Procédé selon la revendication 1 caractérisé en ce que les hydroxydes de métaux alcalins ou les métaux alcalins sont mis en jeu en des quantités d'au moins 1 équivalent molaire par rapport au substrat à réduire.

5. Procédé selon la revendication 1 caractérisé en ce que l'alcool est l'isopropanol ou le butanol secondaire.

6. Procédé selon la revendication 1 caractérisé en ce que la réduction est effectuée en présence d'un benzodiol non substitué ou substitué ou d'une naphtoquinone non substituée ou substituée.

7. Procédé selon la revendication 1 caractérisé en ce que la réduction est effectuée dans un milieu qui ne contient pas d'eau ou qui en contient au plus 15 % en volume.